(19) Europäisches Patentamt
European Patent Office
Office européen des brevets

(11) Publication number: 0 254 364 B1

(12) # EUROPEAN PATENT SPECIFICATION

(45) Date of publication of patent specification: 11.09.91 Bulletin 91/37

(51) Int. Cl.$^5$: C07D 233/90, A01N 43/50

(21) Application number: 87201360.2

(22) Date of filing: 14.07.87

(54) **Imidazole derivative.**

The file contains technical information submitted after the application was filed and not included in this specification

(30) Priority: 21.07.86 GB 8617791

(43) Date of publication of application: 27.01.88 Bulletin 88/04

(45) Publication of the grant of the patent: 11.09.91 Bulletin 91/37

(84) Designated Contracting States: AT BE CH DE ES FR GB GR IT LI LU NL SE

(56) References cited:
EP-A- 0 191 514
DE-A- 3 217 094

(73) Proprietor: SHELL INTERNATIONALE RESEARCH MAATSCHAPPIJ B.V.
Carel van Bylandtlaan 30
NL-2596 HR Den Haag (NL)

(72) Inventor: Pettman, Roger Bruce
5 Glovers Crescent
Sittingbourne Kent (GB)
Inventor: Wells, Nicholas Secker
38 Agnew Road
Forest Hill London SE23 (GB)

(74) Representative: Bennett, David Arthur Horder et al
4, York Road
London SE1 7NA (GB)



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention relates to an imidazole derivative, a process for its preparation, and to the use of that derivative as a fungicide.

DE-A-3217094 (Hoechst) discloses the preparation of various imidazole-5-carboxylic acid derivatives of formula

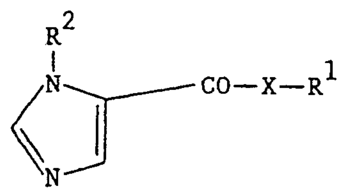

wherein $R^2$ is a phenyl or benzhydryl group optionally substituted by one or more substituents selected from halogen, $C_{1-4}$ alkoxy and $C_{1-4}$ alkyl moieties, X is 0, S or $-NR^1-$ and $R^1$ is H, phenyl, $C_{2-6}$ alkenyl, optionally substituted $C_{1-12}$ alkyl or, when X is 0 or S, a metal cation or ammonium. These derivatives are described as having fungicidal, herbicidal and plant-growth regulant activity, and the compounds wherein $R^2$ is an optionally substituted 2,6-dialkylphenyl group are claimed to be novel.

Applicants European Patent Application No.86200092.4 (EP-A-191514) discloses imidazole-5-carboximidates of general formula

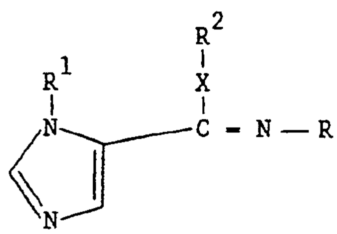

wherein R represents an optionally substituted phenyl group, $R^1$ represents an optionally substituted alkyl, cycloalkyl, alkenyl, aryl or aralkyl group, $R^2$ represents an optionally substituted alkyl, cycloalkyl, cycloalkylalkyl, alkenyl, aryl or aralkyl group, and X represents an oxygen or sulphur atom or a group $-NR^3-$, where $R^3$ represents a hydrogen atom or an alkyl group or $R^2$ and $R^3$ together with the interjacent nitrogen atom represent a 5- or 6- membered saturated or unsaturated heterocyclic ring optionally containing one or two further heteroatoms.

It has now been found that one compound within that general formula, but not specifically disclosed in EP-A-0191514, is of particular interest as a fungicide. Accordingly, the present invention provides the imidazole carboximidate of formula (I)

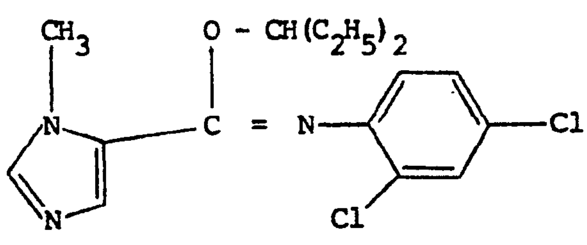

(I)

The present invention also provides a process for the preparation of the compound of formula I by reacting a compound of formula (II)

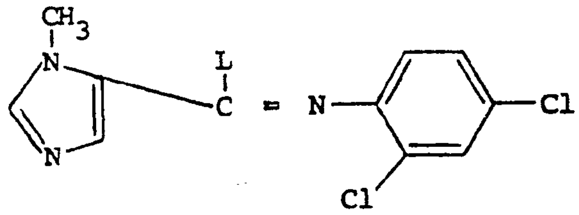

(II)

in which L represents a leaving group, with pentan-3-ol in the presence of a base, preferably a solution of sodium metal in pentanol.

The leaving group L is conveniently a halogen, preferably a chlorine or bromine atom or an alkoxy, preferably a $C_{1-4}$ alkoxy and, most preferably, a methoxy group.

The above process may be effected in the absence of an additional inert solvent e.g. when the pentanol is in excess and the excess acts as a solvent. Alternatively an additional, inert solvent may be present. Suitable solvents include dimethoxyethane, dimethylsulphoxide, N,N-dimethylformamide and tetrahydrofuran. Dimethoxyethane and dimethylsulphoxide have been found to be very suitable.

Compounds of formula (II) in which L represents a halogen atom may conveniently be prepared by reacting a halogenating agent with a compound of formula (III)

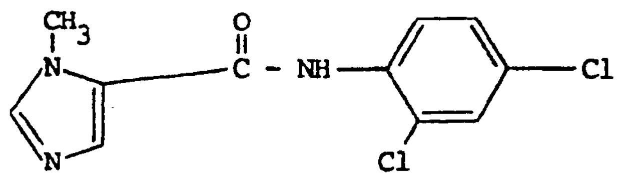

(III)

Suitable halogenating agents include thionyl chloride, thionyl bromide, phosphorous pentachloride, phophorous trichloride and phosphorous tribromide. Such reaction may if desired be effected in the presence of an inert solvent such as toluene, benzene, diethyl ether or tetrahydrofuran.

Compounds of formula (II) in which L represents an alkoxy group may conveniently be prepared from the corresponding compound of formula (II) in which L represents a halogen atom by reaction with an appropriate sodium alkoxide. Such reaction may, if desired, be carried out in the presence of an inert solvent such as toluene or an alcohol, such as methanol.

Compounds of formula (III) are either known compounds or can be prepared by processes analogous to known processes, e.g. to processes described in R.G. Jones, J.Am.Chem.Soc. 71 (1949), 644.

Further in accordance with the invention there is provided a fungicidal composition which comprises a carrier and, as active ingredient, an imidazole derivative of formula I as defined above.

A composition according to the invention preferably contains from 0.5 to 95% by weight of active ingredient.

A carrier in a composition according to the invention is any material with which the active ingredient is formulated to facilitate application to the locus to be treated, which may for example be a plant, seed or oil, or to facilitate storage, transport or handling. A carrier may be a solid or a liquid, including a material which is normally gaseous but which has been compressed to form a liquid, and any of the carriers normally used in formulating fungicidal compositions may be used.

Suitable solid carriers include natural silicas such as diatomaceous earths; magnesium silicates, for example talcs; magnesium aluminum silicates, for example attapulgites and vermiculites; aluminium silicates, for example kaolinites, montmorillonites and micas; calcium carbonate; calcium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes, for example beeswax, paraffin wax, and chlorinated mineral waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Fungicidal compositions are often formulated and transported in a concentrated from which is subsequently diluted by the user before application. The presence of small amounts of a carrier which is a surface-active agent facilitates this process of dilution. Thus preferably at least one carrier in a composition according to the invention is a surface-active agent. For example, a composition may contain at least two carriers, et least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as sodium dodecyl benzene sulponate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75%w of active ingredient and usually contain, in addition to solid inert carrier, 3-10%w of a dispersing agent and, where necessary, 0-10%w of stabiliser(s) and/or other additives such as penetrants or stickers. Dusts are usually formulated as a dust concentrate having a similar composition to that of a wettable powder but without a dispersant, and may be diluted in the field with further solid carrier to give a composition usually containing $\frac{1}{2}$-10%w of active ingredient. Granules are usually prepared to have a size between 10 and 100 BS mesh (1.676-0.152mm), and may be manufactured by agglomeration or impregnation techniques. Generally, granules will contain $\frac{1}{2}$-25%w active ingredient and 0- 10%w of additives such as stabilisers, slow release modifiers and binding agents. Emulsifiable concentrates usually contain, in addition to a solvent and, when necessary, co-solvent, 1-50%w/v active ingredient, 2-20%w/v emulsifiers and 0-20%w/v of other additives such as stabilisers penetrants and corrosion inhibitors. Suspension concentrates are usually compounded so as to obtain a stable, non-sedimenting flowable product and usually contain 10-75%w of active ingredient, 0.5%-15%w of dispersing agents, 0.1-10%w of suspending agents such as protective colloids and thixotropic agents, 0-10%w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as antifreeze agents for water.

Aqueous dispersions and emulsions, for example compositions obtained by diluting a wettable powder or a concentrate according to the invention with water, also lie within the scope of the present invention. The said emulsions may be of the water-in-oil or of the oil-in-water type, and may have a thick 'mayonnaise'-like consistency.

The compositions may also contain other ingredients, for example other compounds possessing pesticidal, especially insecticidal, acaricidal, herbicidal or fungicidal, properties

Of particular interest in enhancing the duration of the protectant activity of the compounds of this invention is the use of a carrier which will provide a slow release of the fungicidal compounds into the environment of the plant which is to be protected. Such slow-release formulations could, for example, be inserted in the soil adjacent to the roots of a vine plant, or could include an adhesive component enabling them to be applied directly to the stem of a vine plant.

The invention still further provides the use as a fungicide of a carboximidate of the general formula (I) as defined above, and a method for combating fungus which comprises treating plants subject to or subjected to fungal attack, seeds of such plants or the medium in which such plants are growing or are to be grown, with such a derivative.

The present invention is of wide applicability in the protection of crop plants against fungal attack. Typical crops which may be protected include vines, grain crops such as wheat and barley, rice and beans. The duration of protection is normally dependent on the individual compound selected, and also a variety of external factors, such as climate, whose impact is normally mitigated by the use of a suitable formulation. Application rates may typically be in the range 0.1 to 10 kg active ingredient per hectare (kg/ha), preferably 0.1 to 1 kg/ha.

The invention will be further understood from the following Examples.

EXAMPLE 1

Preparation of 1-ethylpropyl-N-(2,4-dichlorophenyl)-1-methylimidazole-5-carboximidate

N-(2,4-dichlorophenyl)-1-methylimidazole-5-carboxamide (2.0g) was refluxed in thionyl chloride (50ml) for 2 hours. Excess thionyl chloride was evaporated off and the residue suspended in dimethoxyethane (25ml). A solution of sodium (0.4g) in pentan-3-ol (35ml) was added to the solution and the reaction mixture refluxed for 18 hours. After cooling, the solvent was evaporated off under reduced pressure and the residue taken up in chloroform, washed with water and dried with magnesium sulphate. The chloroform was evaporated off under reduced pressure to give an oil, which was chromatographically purified to yield the desired product as a pale yellow oil.

| | | | | |
|---|---|---|---|---|
| Analysis | Found: | C 56.5; | H 5.7; | N 12.3 |
| $C_{16}H_{19}Cl_2N_3O$ | requires: | C 56.5; | H 5.6; | N 12.3 |

EXAMPLE 2

Preparation of 1-ethylpropyl-N-2,4-dichlorophenyl)-1-methylimidazole-5-carboximidate

(a) Preparation of methoxy-N-(2,4-dichlorophenyl)-1-methylimidazole-5-carboximidate

Thionyl chloride (22.5ml, 0.3 mol) in toluene (50ml) was added over a period of 1 hour to a slurry of N-(2,4-dichlorophenyl)-1-methylimidazole-5-carboxamide (54g, 0.2 mol)in refluxing toluene (450ml) and the resultant mixture strirred under reflux overnight. 250ml of toluene were distilled off, 250ml of toluene were then added and a further 300ml of toluene were then distilled off. A solution of sodium methoxide in methanol was prepared by adding sodium metal (13.8g, 0.6 mol) to methanol (200ml) and the resultant solution was then cooled to -5°C. The cooled sodium methoxide/methanol solution was then added to the slurry, which had also cooled to -5°C, and the resultant mixture stirred at room temperature overnight. 250ml of water were then added and the organic layer was then separated, washed twice with water and dried with anhydrous magnesium sulphate. The organic fraction was then rotary evaporated to dryness and triturated with hexane to give methoxy-N-(2,4-dichlorophenyl)-1-methylimidazole-5-carboximidate (52.6g).

(b) Preparation of 1-ethylpropyl-N-(2,4-dichlorophenyl)-1-methylimidazole-5-carboximidate

To a solution of methoxy-N-(2,4-dichlorophenyl)-1-methylimidazole-5-carboximidate (993g, 3.5 mol) obtained as described in (a) in pentan-3-ol (3 litres, 27.5 mol) at gentle reflux were added small portions of sodium (about 1.5g) until reaction was initiated. The reaction mixture was stirred under reflux for 8 hours with slow removal of the methanol formed until a 99% conversion had been achieved as indicated by gas liquid chromatography. A further quantity of sodium (about 0.5g) was then added and about 100ml of pentan-3-ol was then distilled off at atmospheric pressure. The reaction mixture was then rotary evaporated to dryness, dissolved in hexane (about 3 litres), washed twice with water and dried with anhydrous magnesium sulphate. Further rotary evaporation and degassing yielded 1-ethylpropyl-N-(2,4-dichlorophenyl)-1-methylimidazole-5-carboximidate (1116g).

EXAMPLE 3

The fungicidal activity of the compound of the invention was investigated by means of the following tests.

(a) Direct protectant activity against vine grey mould (Botrytis cinerea; Bcp)

The test is a direct protectant one, using a foliar spray. The lower surfaces of leaves of whole vine plants (cv Cabernet Sauvignon) are sprayed with a solution of active material in 1:1v/v water/acetone containing 0.04%w "Triton X-155" (trade mark) (octylphenol polyoxyethylene surfactant), at a dosage of 1 kilogram of active material per hectare using a track sprayer which delivers 620 l/ha, and after a subsequent 24 hours under normal glasshouse conditions the lower surfaces of the leaves are inoculated by spraying with an aqueous solution containing $10^5$ conidia/ml. The inoculated plants are kept for 24 hours in a high humidity compartment, 5 days under normal glasshouse conditions and then returned for a further 24 hours to high humidity. Assessment

is based on the percentage of leaf area covered by sporulation compared with that on control leaves.

(b) Activity against wheat leafspot (Leptosphaeria nodorum; Ln.)

The test is a direct antisporulant one, using a foliar spray. Leaves of wheat plants (cv Mardler), at the single leaf stage, are inoculated by spraying with an aqueous suspension containing 8 x $10^5$ spores/ml. The inoculated plants are kept for 24 hours in a high humidity compartment prior to treatment. The plants are sprayed at a dosage of 1 kg. of active material per hectare using a track sprayer as described under (a). After drying, the plants are kept for 5 days under normal glasshouse conditions, followed by assessment. Assessment is based on the percentage of leaf area covered by sporulation compared with that on leaves of control plants.

(c) Activity against barley powdery mildew (Erysiphe graminis f.sp. hordei; Eg)

The test is a direct antisporulant one, using a foliar spray. Leaves of barley seedlings, cultivar Golden Promise, are inoculated by dusting with mildew conidia one day prior to treatment with the test compound. The inoculated plants are kept overnight at glasshouse ambient temperature and humidity prior to treatment. The plants are sprayed at a dosage of 1kg. of active material per hectare using a track sprayer as described under (a). After drying, plants are returned to a compartment at ambient temperature and humidity for up to 7 days, followed by assessment. Assessment is based on the percentage of leaf area covered by sporulation compared with that on leaves of control plants.

(d) Activity against broad bean rust (Uromyces fabae Uf)

The test is direct antisporulant one using foliar spray. Pots containing 1 plant per pot were inoculated by spraying an aqueous suspension, containing 5x$10^4$ spores/ml plus a little "Triton X-155", onto the upper surface of each leaf 20-24 hours before treatment with test compound. The inoculated plants were kept overnight in a high humidity compartment, dried at glass-house ambient temperature and then sprayed, on the leaf upper surface, at a dosage of 1kg/ha of active material using a track sprayer as described under (a). After treatment the plants were kept at glass-house temperature and assessment made 11-14 days after treatment. Symptoms are assessed on the relative density of sporulating pustules per plant compared with that on control plants.

(e) Activity against wheat eyespot in-vitro (Pseudocercosporella herpotrichoides; PhI)

This test measure the in vitro activity of compounds against the fungus causing wheat eyespot.

The Test Compound is dissolved or suspended in acetone and is added to molten half strength Potato Dextrose Agar to give a final concentration of 100ppm compound and 3.5% aceetone. After agar has set, plates are inoculated with 6mm diameter plugs of agar/mycelium taken from a 14 day old culture of P. herpotrichoides.

Plates are incubated at 20°C for 12 days and radial growth from the inoculation plug is measured.

The extent of disease control in all the above tests is expressed as a rating compared with a diluent-sprayed control according to the criteria:-

0 = less than 50% disease control
1 = about 50-80% disease control
2 = greater than 80% disease control

Results of the above tests are given in Table I following:

Table I

Fungicidal Activity

| Bcp | Ln | Eg | Uf | PhI |
|---|---|---|---|---|
| 2 | 2 | 2 | 2 | 2 |

## Claims

1. An imidazole derivative of the formula (I)

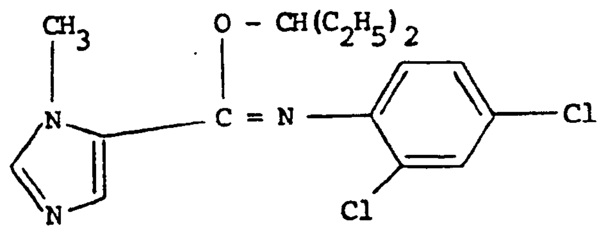

(I)

2. A process for the preparation of an imidazole derivative of formula I as defined in Claim 1 which comprises reacting a compound of formula (II):

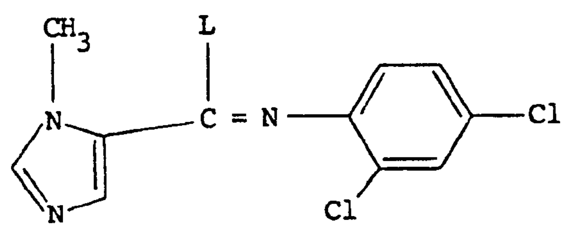

(II)

in which L represents a leaving group, with pentan-3-ol in the presence of a base.

3. A process according to Claim 2 wherein the compound of formula (II) is reacted with a solution of sodium metal in pentanol.

4. A process according to Claim 2 or Claim 3 in which L represents a halogen or an alkoxy group.

5. A process according to Claim 4 in which L represents a chlorine atom, a bromine atom or a methoxy group.

6. A fungicidal composition which comprises at least one carrier and, as active ingredient, an imidazole derivative of formula (I) as defined in Claim 1.

7. A composition according to Claim 6 which comprises at least two carriers, at least one of which is a surface-active agent.

8. A method of combating fungus which comprises treating plants subject or subjected to fungal attack, seeds of such plants or the medium in which the plants are growing or are to be grown with an imidazole derivative of the formula (I) as defined in Claim 1.

## Patentansprüche

1. Imidazolderivat der Formel (I)

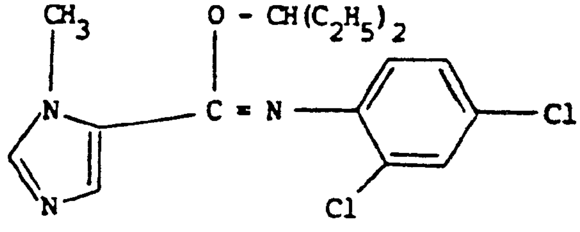

(I).

2. Verfahren zur Herstellung eines Imidazolderivats der Formel (I), wie in Anspruch 1 definiert, welches ein Umsetzen einer Verbindung der Formel (II)

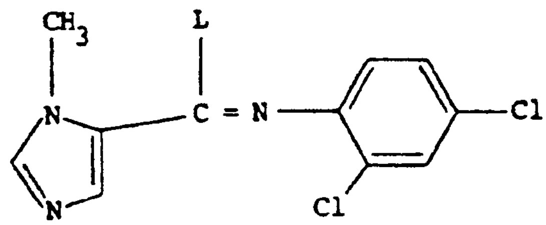

(II),

worin L eine Leavinggruppe darstellt, mit Pentan-3-ol in Gegenwart einer Base umfaßt.

3. Verfahren nach Anspruch 2, worin die Verbindung der Formel (II) mit einer Lösung von Natriummetall in Pentanol umgesetzt wird.

4. Verfahren nach Anspruch 2 oder 3, worin L ein Halogen oder eine Alkoxygruppe darstellt.

5. Verfahren nach Anspruch 4, worin L ein Chloratom, ein Bromatom oder eine Methoxygruppe bedeutet.

6. Fungizide Zusammensetzung, welche wenigstens einen Träger und als wirksamen Bestandteil ein Imidazolderivat der Formel (I), wie in Anspruch 1 definiert, umfaßt.

7. Zusammensetzung nach Anspruch 6, welche wenigstens 2 Träger umfaßt, von denen wenigstens einer ein oberflächenaktives Mittel ist.

8. Verfahren zur Bekämpfung von Pilzen, welches ein Behandeln von Pflanzen, die einem Pilzbefall unterliegen oder unterworfen sind, von Sämlingen und Samen solcher Pflanzen oder des Mediums, worin die Pflanzen wachsen oder gezogen werden sollen, mit einem Imidazolderivat der Formel (I), wie in Anspruch 1 definiert, umfaßt.

## Revendications

1. Un dérivé d'imidazole de la formule (I)

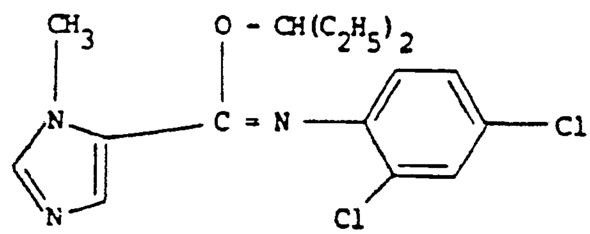

(I)

2. Un procédé pour la préparation d'un dérivé d'imidazole de formule I tel que défini dans la revendication 1, qui comprend la réaction d'un composé de formule (II)

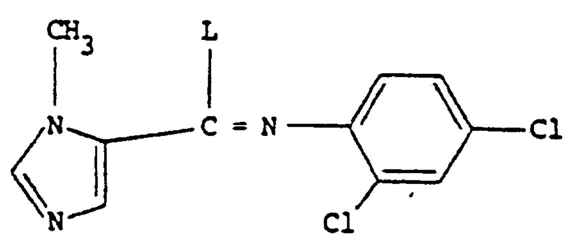

(II)

où L représente un groupe partant, avec du pentan-3-ol en présence d'une base.

3. Un procédé selon la revendication 2, dans lequel le composé de formule (II) est mis à réagir avec une solution de sodium métallique dans du pentanol.

4. Un procédé selon la revendication 2 ou la revendication 3, dans lequel L représente un halogène ou un groupe alcoxy.

5. Un procédé selon la revendication 4, dans lequel L représente un atome de chlore, un atome de brome ou un groupe méthoxy.

6. Une composition fongicide qui comprend au moins un véhicule et, comme ingrédient actif, un dérivé d'imidazole de formule (I) tel que défini dans la revendication 1.

7. Une composition selon la revendication 6, qui comprend au moins deux véhicules, dont au moins un est un agent tensio-actif.

8. Une méthode de lutte contre les champignons, qui comprend le traitement de plantes sensibles ou soumises à une attaque fongique, de semences de telles plantes ou du milieu dans lequel les plantes poussent ou doivent pousser, avec un dérivé d'imidazole de la formule (I) tel que défini dans la revendication 1.